# NEUE EUROPÄISCHE PATENTSCHRIFT

(11) **EP 0 293 708 B2**
(45) Veröffentlichungstag und Bekanntmachung des Hinweises auf die Entscheidung über den Einspruch: **26.04.1995**
(45) Hinweis auf die Patenterteilung: 03.07.1991
(21) Anmeldenummer: 88108211.9
(22) Anmeldetag: 21.05.1988
(51) Int. Cl.: A61K 31/505, A61K 9/08, A61K 47/02

(54) **Stabile 5-Fluorouracil-Formulierungen**
Stable formulations of 5-fluorouracil
Formulations stables de 5-fluorouracile

(30) Priorität: 03.06.1987 US 57149
(43) Veröffentlichungstag der Anmeldung: 07.12.1988
(73) Patentinhaber: F. HOFFMANN-LA ROCHE AG, 4002 Basel (CH)
(72) Erfinder: Johnson, James B., Upper Montclair, N.J. 07043 (US)
(74) Vertreter: Notegen, Eric-André

(56) Entgegenhaltungen:
- FR-A- 2 386 310
- MICROCHEMICAL JOURNAL, Band 22, 1977, Seiten 70-84, Academic Press, New York, US; H. TOMANKOVA et al.: "Study of the stability of pyrimidine series cytostatics, ftorafur and fluorouracil injections"
- CHEMICAL ABSTRACTS, Band 89, 1978, Seite 308, Zusammenfassung Nr. 95005e, Columbus, Ohio, US; & JP-A-78 18 722 (TEIKOKU CHEMICAL INDUSTRY CO., LTD) 21-02-1978
- CHEMICAL ABSTRACTS, Band 83, 1975, Seite 275, Zusammenfassung Nr. 168466u, Columbus, Ohio, US; & JP-A-75 100 219 (TOYAMA CHEMICAL CO., LTD) 08-08-1975
- Martindale, THE EXTRA PHARMACOPEIA, London 1982, Seite 211
- Pharmacy Update, June 10 1991, p. 17
- The United States Pharmacopeia, 21st. Revision, 01-01-1985, p.441- 442
- Supplement 3 to the United States Pharmacopeia, 21st.Revision, 1-11-85, p. 1993
- British Pharmacopeia 1980, Addendum 1983, page 279

## Beschreibung

5-Fluorouracil (5FU) ist ein fluoriertes Pyrimidin, das antimetabolische Wirkungen entfaltet und ein bekannter antineoplastischer Wirkstoff ist.

5FU wird seit langer Zeit als Mittel zur Palliativbehandlung verschiedener Carcinoma einschliesslich der Carcinoma des Colons, des Rektums, der Brust, des Magens und der Pankreas verwendet. Es wird häufig bei Patienten verwendet, welche operativ oder mittels anderer Methoden für nicht heilbar gehalten werden.

Es wird angenommen, daß diese Verbindung die Synthese der DNA beeinträchtigt und, in einem geringeren Ausmaß die Bildung der RNA hemmt. Da die DNA und die RNA für das Zellwachstum und die Zellteilung unentbehrlich sind, könnte der Effekt von 5FU darin bestehen, dass es ein Thymin-Defizit bewirkt, welches zu einem unkontrollierten Wachstum und zum Tod der Zelle führt. Die Auswirkungen des Defizites an DNA und RNA sind insbesondere bei Zellen, wie Carcinomazellen, welche sehr rasch wachsen, stark ausgeprägt.

5FU ist ein im Handel erhältliches Produkt und wird gewöhnlich in Form von 10 ml-Ampullen mit einer Konzentration von 50 mg/ml für die intravenöse Injektion bereitgestellt. Die bekannten 5FU-Formulierungen sind farblose bis schwach gelbe wässrige Lösungen mit einem pH von etwa 8,8 (vgl. Hartindale, The Extra Pharmacopoeia, The Pharmaceutical Press, London, 1982). Die bekannten Fluorouracil-Formulierungen sind kälteempfindlich und müssen bei Temperaturen oberhalb etwa 15° C (60° F) aufbewahrt werden, um eine Ausfällung zu verhindern. Während des Transportes vom Hersteller zum Verbraucher werden diese Formulierungen häufig Temperaturen ausgesetzt, welche unterhalb dieses Bereiches liegen. Wenn die Ampullen dann den Bestimmungsort erreichen, enthalten sie das 5FU in Form einer Ausfällung bestehend aus großen Agglomeraten. In dieser Form ist das Produkt für die Weiterverwendung ungeeignet.

Die Bildung von Niederschlägen bei niedrigen Temperaturen während Transporten im Winter ist ein statistisches Phänomen und tritt häufig auf. Dies hat die unangenehme Folge, daß der Niederschlag unter Erwärmen auf 60° C (140° F) unter starkem Schütteln wieder aufgelöst werden muss. Die Lösung muß dann langsam auf Körpertemperatur abgekühlt werden, bevor sie verwendet werden kann.

Es war bisher nicht möglich, eine 5fu-Formulierung in einem verschlossenen Behälter, welche für die Verabreichung einer injizierbaren Einheitsdosis geeignet ist, zur Verfügung zu stellen, aus welchen das 5FU nicht ausfällt, wenn sie niedrigen Temperaturen ausgesetzt werden, was häufig bei Transporten auftritt. Bei Formulierungen in Form von Fläschchen ist die Ausfällung bei niedrigen Temperaturen ein besonderes Problem. In diesem Fall können Ausfällungen bereits bei Temperaturen und Bedingungen auftreten, bei welchen die entsprechenden Ampullen-Formulierungen stabil bleiben.

Demgemäss bestand schon seit längerer Zeit ein Bedürfnis für eine stabile 5FU-Formulierung, welche für die Aufbewahrung in einem verschlossenen Behälter und für die Verabreichung einer injizierbaren Einheitsdosis geeignet ist und aus welchem das 5FU bei Temperaturen unterhalb von 15° C (60° F), welche häufig während Transporten auftreten, nicht ausfällt.

In Chemical Abstracts 83, 168466u (1975) wird die Stabilisierung von wässrigen 5-FU-Lösungen durch Zugabe von Aethylenglycol, Propylenglycol oder Polyäthylenglycol beschrieben.

Die vorliegende Erfindung betrifft 5FU-Präparate, welche bei Temperaturen bis zu 0° C (32° F) stabil bleiben, wenn sie in verschlossenen Behältern aufbewahrt werden, welche für die Verabreichung einer injizierbaren Einheitsdosis geeignet sind.

Die vorliegende Erfindung betrifft ebenfalls ein Verfahren zur Herstellung von 5FU-Präparaten, welche bei Temperaturen bis zu 0° C (32° F) stabil bleiben, wenn sie in verschlossenen Behältern aufbewahrt werden, welche für die Verabreichung einer injizierbaren Einheitsdosis geeignet sind.

Die erfindungsgemässen 5FU-Präparate enthalten eine Lösung von 5FU und eine Base. Die 5FU-Lösung können mittels herkömmlicher, dem Fachmann bekannten Methoden hergestellt werden. Es wird dann eine Base dazugegeben, so dass der pH des fertigen Präparates 9,2 ist.

Besonders bevorzugt ist eine mittels herkömmlicher Methoden hergestellte 5FU-Lösung, zu welcher man eine Base, insbesondere Natriumhydroxid, dazugegeben hat, so daß der pH des fertigen Präparates 9,2 beträgt und die Konzentration an 5FU 50 mg/ml beträgt.

Diese Präparate bleiben bei Temperaturen bis zu 0° C (32° F) stabil, wenn der für die Verabreichung einer injizierbaren Einheitsdosis geeignete, verschlossene Behälter eine Ampulle ist. Diese Präparate bleiben auch stabil bis zu Temperaturen von 0° C (32° F) in Fläschchen, welche mit den nachfolgend aufgeführten Gummiverschlüssen versehen sind, welche ihrerseits mittels an sich bekannter Standard-Natriumphosphat-Waschmethoden gewaschen worden sind:
1. Nitril-Butadien-Gummiverschlüsse;
2. Halo-Butadien-Gummiverschlüsse;
3. Jeder Gummiverschluss, welcher mit einem fluorierten Kohlenwasserstoff-Polymer überzogen ist.

Mit Nitril-Butadien-Gummiverschluss sind Gummiverschlüsse aus Nitril-Butadien-Elastomeren, welche mit einem inerten Mineral verstärkt und mit einem organischen Peroxid vulkanisiert worden sind (wie West® 2212), gemeint.

Mit Halo-Butadien-Gummiverschluss sind Gummiverschlüsse aus Halo-Butadien-Elastomeren, welche mit einem inerten Mineral verstärkt und mit einem phenolischen Harz vulkanisiert worden sind (wie West® PH 703/VII), gemeint.

Mit fluorierten Kohlenwasserstoff-Polymeren sind alle Zusammensetzungen gemeint, welche fluorierte Kohlenwasserstoff-Polymere enthalten und als Ueberzugsmaterial verwendet werden.

Alle West-Produkte werden durch die West Company, Phoenixville, PA 19469, hergestellt.

Die erhaltenen 5FU-Präparate stellen in Bezug auf erhöhte Resistenz gegen Ausfällung gegenüber den bekannten Präparaten eine signifikante Verbesserung dar.

Die vorliegende Erfindung wird im Zusammenhang mit dem folgenden Beispiel näher erläutert, das allerdings lediglich der Illustration dient.

### Beispiel

West® PH 703/VII- oder West® 2212-Gummiverschlüsse, welche durch West Company hergestellt worden sind, werden gemäss dem nachfolgenden Verfahren gewaschen.
1. Lege die zu waschenden Gummiverschlüsse in ein sauberes Gefäss aus rostfreiem Stahl oder aus Glas. Ueberdecke die Verschlüsse mit einer 1-proz. Lösung von Tetranatriumpyrophosphat in destilliertem Wasser.
2. Autoklaviere den Behälter während einer halben Stunde bei 121° C.
3. Dekantiere die noch heisse Flüssigkeit von den Verschlüssen ab und spüle die Verschlüsse unter Rühren gründlich mit destilliertem Wasser.
4. Tauche die Verschlüsse in destilliertes Wasser und autoklaviere während einer halben Stunde bei 121° C.
5. Wiederhole die Stufen 3 und 4.
6. Prüfe das Spülwasser auf Klarheit. Falls das Spülwasser nicht klar ist, wiederhole das Autoklavieren mit destilliertem Wasser bis das abdekantierte Wasser klar erscheint.
7. Sterilisiere die gewaschenen Verschlüsse durch Autoklavieren in geeigneten, luftdurchlässigen Behältern (z.B. Autoklavierbeutel) während 45 Minuten bei 121° C. Nach Beendigung dieser Sterilisation sollte ein Trocknungszyklus von wenigstens 5 Minuten angewendet werden, um die Trockenheit der sterilen Verschlüsse sicherzustellen. Die Verschlüsse sollten innerhalb von 48 Stunden nach der Sterilisation verwendet werden.

Das 5FU-Präparat wird ausgehend von einer 5FU-Lösung, welche nach herkömmlichen, dem Fachmann bekannten Methoden hergestellt worden ist, hergestellt. Diese Lösung wird dann mit Natriumhydroxid auf 9,2 gebracht. Das Volumen wird mit Wasser so eingestellt, daß die Konzentration an 5FU am Ende 50 mg/ml beträgt.

Das Präparat wird anschliessend in Fläschchen abgefüllt, welche mit den gewaschenen Gummiverschlüssen verschlossen werden.

Die Tabelle I illustriert Vergleichsversuche der anmeldegemässen 5FU-Präparate in Fläschchen- und Ampullen-Formulierungen.

**Tabelle I**

| Ausfällungsstudie bei gekühlter Aufbewahrung (5°C) | | | | |
|---|---|---|---|---|
| Ampulle/Fläschchen | Tag an welchem Ausfällung zuerst beobachtet wurde | | Ausgefallene Einheiten nach 5 Wochen | |
| | Stationär | Schütteln | Stationär | Schütteln |
| | | | pH 9,2 (kein 5FU-Ueberschuß) | |
| Ampulle⁽¹⁾ | - | - | 0/45 | 0/25 |
| Fläschchen - West® 2212 | - | - | 0/25 | 0/25 |
| Fläschchen -West PH 703/VII | - | - | 0/25 | 0/25 |

| | | | pH 8,8 (kein 5FU-Ueberschuß) | |
|---|---|---|---|---|
| Ampulle⁽¹⁾ | 1 | 3 | 6/50 | 10/50 |
| Fläschchen - West® 2212 | 10 | 3 | 10/25 | 17/25 |
| Fläschchen -West PH 703/VII | 7 | 2 | 12/25 | 23/25 |

| | | | pH 8,8 (5% 5FU-Ueberschuss) | |
|---|---|---|---|---|
| Ampulle | 1 | 3 | 19/25 | 18/25 |

| | | | | |
|---|---|---|---|---|
| (1) Die Daten sind das Ergebnis zweier Studien. Als Kontrolle für die mit West® PH 703/VII- und West® 2212-Verschlüssen versehenen Fläschchen wurden Ampullen verwendet. | | | | |

## Patentansprüche

1. 5-Fluorouracil-Präparate mit einem pH vou 9,2, welche im wesentlichen aus einer Lösung von 5-Fluorouracil und einer Base bestehen.

2. Präparate nach Anspruch 1, worin die Konzentration von 5-Fluorouracil 50 mg/ml beträgt.

3. Präparate nach Anspruch 1 oder 2, worin die Base Natriumhydroxid ist.

4. Präparate nach Anspruch 1, worin die Konzentration an 5-Fluorouracil 50 mg/ml und der pH 9,2 betragen und die Base Natriumhydroxid ist.

5. Eine für die Verabreichung einer injizierbaren Einheitsdosis von 5-Fluorouracil geeignete pharmazeutische Dosierungsform, bestehend aus einem verschlossenen Behälter enthaltend ein Präparat nach einem der Ansprüche 1-4.

6. Eine pharmazeutische Dosierungsform nach Anspruch 5, dadurch gekennzeichnet, daß der verschlossene Behälter eine Ampulle ist.

7. Eine pharmazeutische Dosierungsform nach Anspruch 5, dadurch gekennzeichnet, daß der verschlossene Behälter ein Glasfläschchen ist, das mit einem Gummiverschluss ausgewählt aus der Gruppe bestehend aus Nitril-Butadien-Gummiverschüssen. Halo-Butadien-Gummiverschlüssen und allen Gummiverschlüssen, welche mit einem fluorierten Kohlenwasserstoff-Polymer überzogen sind, welcher mit Natriumphosphat gewaschen worden ist, verschlossen ist.

8. Ein Verfahren zur Herstellung von 5-Fluorouracil-Präparaten nach einem der Ansprüche 1-4, dadurch gekennzeichnet, daß man den pH einer 5 Fluorouracil-Lösung mit einer Base auf 9,2 einstellt und die gewünschte Konzentration an 5-Fluorouracil mit Wasser einstellt.

9. Ein Verfahren zur Herstellung von pharmazeutischen Dosierungsformen nach einem der Ansprüche 5-7, dadurch gekennzeichnet, daß man den pH einer 5-Fluorouracil-Lösung mit einer Base auf 9,2 einstellt, die gewünschte Konzentration an 5-Fluorouracil mit Wasser einstellt und die gewünschte Menge des so erhaltenen Präparates in verschlossene Behälter abfüllt.

## Claims

1. A 5-fluorouracil preparation having a pH of 9.2 which consists essentially of a solution of 5-fluorouracil and a base.

2. A preparation according to claim 1, wherein the concentration of 5-fluorouracil is 50 mg/ml.

3. A preparation according to claim 1 or 2, wherein the base is sodium hydroxide.

4. A preparation according to claim 1, wherein the concentration of 5-fluorouracil is 50 mg/ml and the pH is 9.2 and the base is sodium hydroxide.

5. A pharmaceutical dosage form suitable for administering an injectable unit dose of 5-fluorouracil, consisting of a sealed container containing a preparation according to any one of claims 1-4.

6. A pharmaceutical dosage form according to claim 5, characterized in that the sealed container is an ampoule.

7. A pharmaceutical dosage form according to claim 5, characterized in that the sealed container is a vial which is sealed with a sodium phosphate-washed rubber closure selected from the group consisting of nitrile-butadiene rubber closures, halo-butadiene rubber closures and any rubber closures which are coated with a fluorinated hydrocarbon polymer.

8. A process for the manufacture of 5-fluorouracil preparations according to any one of claims 1-4, characterized by adjusting the pH of a 5-fluorouracil solution to 9.2 with a base and adjusting the desired concentration of 5-fluorouracil with water.

9. A process for the manufacture of pharmaceutical dosage forms according to any one of claims 5-7, characterized by adjusting the pH of a 5-fluorouracil solution to 9.2 with a base, adjusting the desired concentration of 5-fluorouracil with water and filling the desired amount of the thus-obtained preparation into a sealed container.

## Revendications

1. Compositions de 5-fluorouracile ayant un pH de 9.2, et qui consistent essentiellement en une solution de 5-fluorouracile et d'une base.

2. Compositions selon la revendication 1, dans lesquelles la concentration du 5-fluorouracile est de 50 mg/ml.

3. Compositions selon la revendication 1 ou 2, dans lesquelles la base est l'hydroxyde de sodium.

4. Compositions selon la revendication 1, dans lesquelles la concentration du 5-fluorouracile est de 50 mg/litre et le pH de 9.2, et la base est l'hydroxyde de sodium.

5. Une forme de dosage pharmaceutique convenant pour l'administration d'une dose unitaire injectable de 5-fluorouracile, qui consiste en un récipient fermé contenant une composition selon l'une des revendications 1 à 4.

6. Une forme de dosage pharmaceutique selon la revendication 5, caractérisée en ce que le récipient fermé est une ampoule.

7. Une forme de dosage pharmaceutique selon la revendication 5, caractérisée en ce que le récipient fermé est un petit flacon de verre fermé par un bouchon de caoutchouc choisi parmi les bouchons de caoutchouc nitrile-butadiène les bouchons de caoutchouc d'halogénobutadiènes et tous les bouchons de caoutchouc revêtus par un polymère hydrocarboné et fluoré, qui ont été lavés par du phosphate de sodium.

8. Un procédé pour la préparation des compositions de 5-fluorouracile selon l'une des revendications 1 à 4, caractérisé en ce que l'on règle le pH d'une solution de 5-fluorouracile à un niveau de 9.2 à l'aide d'une base et on règle à la concentration voulue en 5-fluorouracile par de l'eau

9. Un procédé de préparation de formes de dosage pharmaceutiques selon l'une des revendications 5 à 7, caractérisé en ce que l'on règle le pH d'une solution de 5-fluorouracile à un niveau de 9.2 à l'aide d'une base, on règle à la concentration voulue en 5-fluorouracile par de l'eau et on introduit la quantité voulue de la composition ainsi obtenue dans des récipients fermés.
